# EUROPEAN PATENT SPECIFICATION

(11) **EP 0 495 321 B1**
(45) Date of publication and mention of the grant of the patent: **02.10.1996**
(21) Application number: 91400074.0
(22) Date of filing: 15.01.1991
(51) Int. Cl.: A61K 31/27, A61K 9/06

(54) **Rapid miosis with control of intraocular pressure**
Schnelle Miosis mit Kontrolle des Augeninnendruckes
Miosis rapide avec contrôle de la pression intra-oculaire

(43) Date of publication of application: 22.07.1992
(73) Proprietor: McKinzie, James W., Ventura, California 93001 (US)
(72) Inventor: McKinzie, James W., Ventura, California 93001 (US)
(74) Representative: Hirsch, Marc-Roger

(56) References cited:
- CHEMICAL ABSTRACTS, vol. 109, no. 13, 26th September 1988, page 133, abstractno. 105304z, Columbus, Ohio, US; H.M. KOO et al.: "Effects of acetylcholine andcarbachol on rabbit pupil size and corneal endothelium",& K'AT'OLLIK TAEHAK UIHAKPU NONMUNJIP 1988, 41(1), 375-86
- CHEMICAL ABSTRACTS, vol. 74, no. 15, 12th April 1971, page 296, abstract no. 74724p, Columbus, Ohio, US; T.O. McDONALD et al.: "Intraocular safety ofcarbamoylcholine chloride (carbachol) in rabbit eyes",& ANN. OPHTHALMOL. 1970, 2(9), 878-9, 882-3

## Description

### Technical Field

This invention relates to a miotic agent useful in post-operative cataract and intraocular lens surgery and, more particularly, to a combination of miotic agents that provides quick miosis with control of intraocular pressure 24 hours after surgery.

### Background of the Invention

Miotic agents are frequently used by ophthalmologic surgeons during intraocular surgery. The anterior chamber is irrigated with a miotic agent after delivery of the lens in cataract surgery as well as in penetrating keratoplasty, iridectomy and other anterior segment surgery. Prompt miosis is necessary to ensure that a round pupil is obtained after cataract surgery. If any of the iris of the eye is caught in the incision or if a capsular tag is caught in the incision, the pupil will be distorted on the following day. It is easy to miss a capsular tag in the incision since the tag is clear and transparent unless one uses a miotic agent. The other advantages obtained by the use of miotics are the facilitation of post-placed corneal scleral sutures, anterior chamber lens insertion and a decrease in post-operative peripheral anterior synechias. Many surgeons feel that miotic agents help in centering and positioning the intraocular lens implant.

Elevated intraocular pressure (IOP) can interfere with normal functioning and may result in irreversible loss of visual function. Viscoelastic agents such as Healon are often used during lens implantation which can cause elevated IOP with pressure spiking.

With the advent of modern surgical techniques and the trend to "in the bag" placement of posterior chamber IOL's, more and more viscoelastic substances are being used. Increasingly, cataract surgery is being done on an out-patient basis, and the patient returns to the physician's office the following day. Slit lamps and applanation tonometry are handy, and consequently most surgeons are examining their post-operative patients even better than when they were hospitalized. This has improved patient care and, on the other hand, has perhaps resulted in increased awareness of the IOP 20-24 hours after cataract surgery.

Pressure studies have shown that the IOP in the first 24 hours after cataract surgery may be very important. Damage by raising IOP is possible to the optic nerve, the vascular supply within the eye, and the corneal endothelium (15) (7). Consequently, every effort should be made to control the IOP from the very onset of the post-operative period.

Acetylcholine (Miochol) is the most popular miotic agent utilized by ophthalmologic surgeons. Miochol provides quick miosis (within minutes). However, it provides very poor control of IOP after several hours, even when pressure control agents such as acetazolamide (Diamox) are utilized. Carbachol (Miostat) does not provide as quick a miosis and is not as widely used. A miotic agent providing quick miosis with control of intraocular pressure 24 hours after surgery is needed.

### Description of the Prior Art

Gormaz (3), in 1962, first reported increases in IOP in the immediate period after cataract extractions. Rich (4) (5) in 1968 and 1969 found a significant rise in IOP was characteristic after cataract surgery. He also showed that α-chymotrypsin was not required to produce this increase. Sodium hyaluronate (Healon) has been implicated as causing a rise in IOP. Olivius and Thornburn (6) have shown that sodium hyaluronate induced increased IOP, and is partially reversible by removal or dilution of the viscoelastic material by irrigation (7).

Several drugs have been used to counteract the increase in IOP associated with cataract surgery. Rich (3) in 1969 demonstrated a lowering of IOP 24 hours after surgery with the use of acetazolamide (Diamox) in high doses during the 24 hours following cataract surgery. However, acetazolamide has some undesirable side effects in some patients. Although Timolol effectively lowered IOP after ICCE (8), it was found to have no effect in acute post-operative pressure elevations following ECCE with IOL and the use of sodium hyaluronate (9). Recently, a simple administration of pilocarpine gel was found to be effective in reducing IOP for the first 24 hours after ECCE with IOL (10). However, patients frequently complained of brow ache the next day. This same group, however, felt that there was a trend in lowering IOP post-operatively using Timolol.

Miotic agents came into use in about 1970, shortly after the onset of ECCE. In 1972, Beasley (11) found that miosis was rapid with both acetylcholine 1% and Carbachol .01%. With Carbachol, miosis extended into the first post-operative day, unlike acetylcholine, where the miotic effect is gone within a very short time. However, Holland, Drance and Schulzer (13), showed that acetylcholine 1% has a more rapid onset of miosis than does Carbachol .01%. Holland, Drance and Schulzer (14) showed that acetylcholine 1%, administered intracamerally during cataract surgery, significantly reduced the IOP at 3 and 6 hours post-operatively but had no effect beyond this time. On the other hand, this same group of investigators showed that Carbachol .01% was highly effective in reducing IOP for at least 24 hours post-operatively, and in reducing the number of patients developing IOP greater than 30mm.Hg.

K'AT'OLLIK TAEHAK UIHAKPU NONMUNIIP 41(1),375-386 (1988) concerns a study of the effects of different concentrations of acetylcholine and carbachol on rabbit pupil size. This document does not describe or suggest the effects of a miotic agent comprising both carbachol and acetylcholine according to the present invention; furthermore there is no indication of the pressure-reducing effect of such a combination.

### List of Cited References

### BIBLIOGRAPHY

(1) U.S. Patent No. 4,459,309
(2) U.S. Patent No. 4,665,094
(3) Gormaz, A.: Ocular Tension After Cataract Surgery. American Journal of Ophthalmology, 43:832, 1962.
(4) Rich, W.J.C.C.: Intraocular Pressures and Wound Closure After Cataract Extraction. Trans. Ophthalmol. Soc., U.K. 88:437, 1968.
(5) Rich, W.J.C.C.: Further Studies on Early Post-operative Ocular Hypertension Following Cataract Extraction. Trans. Ophthalmol. Soc., U.K. 89:639, 1969.
(6) Olivius, E., and Thornburn, W.: Intraocular Pressure After Surgery with Healon. Am. Intraocular Implant Soc. J. 11:480, 1985.
(7) Cherfan, G.M., Rich, W.J. and Wright, G.: Raised Intraocular Pressure and Other Problems with Sodium Hyaluronate and Cataract Surgery. Trans. Ophthalmol. Soc., U.K. 103:277, 1983.
(8) Obstbaum, S.A. and Galin, M.A.: The Effects of Timolol on Cataract Extraction and Intraocular Pressure. Am. J. Ophthalmol. 88:1017, 1979.
(9) Tomoda, T., Tuberville, A.W., and Ward, T.O.: Timolol and Postoperative Intraocular Pressure. Am. Intraocul. Implant Soc. J., 10:180, 1984.
(10) Ruiz, R.S., Wilson, C.A., Musgrove, K.H. and Prager, T.C.: Management of Increased Intraocular Pressure After Cataract Extraction. Am. J. Ophthalmol., 103:487, 1987.
(11) Beasley, H.: Miotics In Cataract Surgery. Arch. Ophthalmol., 88:49, 1972.
(12) Douglas, G.R.: A Comparison of Acetylcholine and Carbachol Following Cataract Extraction. Can. J. Ophthalmol., 8:75, 1973.
(13) Hollands, R.H., Drance, S.M., and Schulzer, M.: The Effect of Intracamerol Carbachol on Intraocular Pressure After Cataract Extraction. Am. J. Ophthalmol., 104:225, 1987.
(14) Hollands, R.H., Drance, S.M., and Schulzer, M.: The Effect of Acetylcholine on Early Postoperative Intraocular Pressure. Am. J. Ophthalmol., 103:749, 1987.
(15) Hayrch, S.S.: Anterior Ischemic Optic Neuropathy, IV, Occurrence After Cataract Extraction. Arch. Ophthalmol., 98:1410, 1980.

### Statement of the Invention

An improved miotic agent is provided in accordance with this invention that provides rapid miosis with 24 hour control of intraocular pressure. The miotic agent of the invention reduces or eliminates the need for IOP control agents such as Diamox and reduces IOP after use of viscoelastic agents such as Healon.

The present invention thus provides :
a product containing a first miotic agent selected from components of the formula: 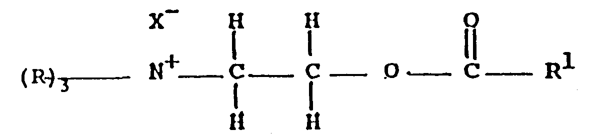 where R and R¹ are lower alkyl groups containing 1 to 5 carbon atoms and X⁻ is an anion such as halo; and
a second miotic agent selected from components of the formula: 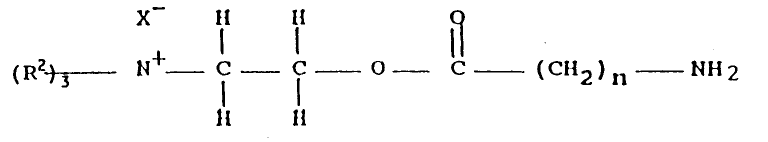 where R² is a low alkyl group of 1-5 carbon atoms, n is an integer from 0-3 and X⁻ is an anion such as halo,
as a combined preparation for simultaneous, separate or sequential use in therapy as a miotic agent controlling intraocular pressure in a mammal.

The miotic agent of the invention resides in the combined use of an acetylcholine type of agent with a carbachol type of agent. The combination provides fast onset of miosis, a prolonged miotic effect, and long-term control of IOP for 24 hours. The IOP is maintained at or below 25 mmHg with very few, if any, pressure spikes in the 24 hour, post-operative period.

The miotic agent of the invention is convenient to use. The agent is safe and effective since it is a combination of two agents approved for use in the same procedure. The miotic agent of the invention will find general use in intraocular surgery and is especially useful in the class of patients who enter the procedure with elevated intraocular pressure, such as glaucoma patients. It will also prove very useful in procedures in which Healon is used to aid in the insertion of an intraocular lens.

The acetylcholine and carbachol materials can be used sequentially or simultaneously. Another aspect of the invention resides in packaging the two agents in a common container having two separate compartments. The agents are combined and dissolved in a common carrier immediately before use.

The present invention thus provides
a unit dosage package (10) of a product capable of quick miosis while controlling intraocular pressure containing:
a first miotic agent selected from components of the formula: 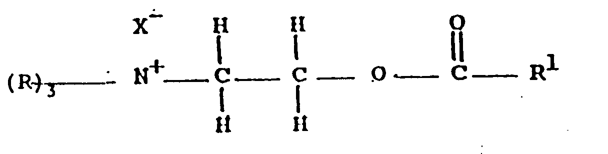 where R and R¹ are lower alkyl groups containing 1 to 5 carbon atoms and X⁻ is an anion such as halo; and
a second miotic agent selected from components of the formula: 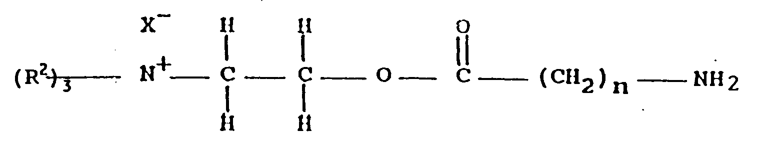 where R² is a low alkyl group of 1-5 carbon atoms, n is an integer from 0-3 and X⁻ is an anion such as halo, which comprises:
a first sterile container (17) containing a unit dosage of the first miotic agent in dry powder form and;
a second sterile container (14) containing a unit dosage of a solution of the second miotic agent in an aqueous saline which is a solvent for the first miotic agent.

These and many other features and attendant advantages of the invention will become apparent as the invention becomes better understood by reference to the following detailed description when considered in conjunction with the accompanying drawings.

### Brief Description of the Drawings

Figure 1a is an enlarged view of a package containing the component of the miotic agent of the invention;
Figure 1b is a view taken along line 1b-1b of Fig. 1a;
Figure 1c is a view similar to Fig. 1b but showing unit dosage and aqueous solution mixed and ready to be withdrawn by a hypodermic needle.
Figure 2 is a set of curves showing pre-operative and post-operative intraocular pressure IOP in a group of patients receiving Miochol with Diamox;
Figure 3 is a set of curves showing pre-operative and post-operative intraocular pressure IOP in a group of patients receiving Miochol without Diamox;
Figure 4 is a set of curves showing pre-operative and post-operative intraocular pressure IOP in a group of patients receiving Miostat with Diamox;
Figure 5 is a set of curves showing pre-operative and post-operative intraocular pressure IOP in a group of patients receiving Miostat without Diamox;
Figure 6 is a set of curves showing pre-operative and post-operative intraocular pressure IOP in a group of glaucoma patients receiving Miochol;
Figure 7 is a set of curves showing pre-operative and post-operative intraocular pressure IOP in a group of glaucoma patients receiving Miostat; and
Figure 8 is a series of bar graphs illustrating the pre- and post-operative IOP in 18 patients administered the combined miotic agent of the invention.

### Detailed Description of the Invention

The first active agent in the miotic composition of the invention is a compound of the formula: 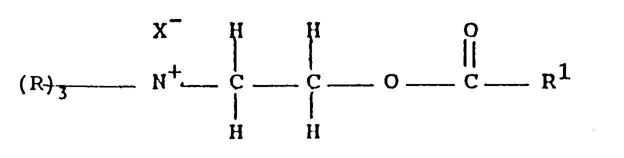 where R and R¹ are lower alkyl groups containing 1 to 5 carbon atoms and X⁻ is an anion such as halo.

The preferred member of this group is acetylcholine where R and R¹ are all methyl and X⁻ is chloro. Acetylcholine is a parasympathemetic agent. It is utilized in concentrations from 0.1 to 5% usually at 1% by weight. Acetylcholine is unstable so it is provided in dry powder form and is mixed with physiologically inert, liquid carrier before use. The dry material can be mixed with an inert lyophilizing material such as mannitol in ratios of 1/1 to 10/1, usually 3/1 by weight. The reconstituted aqueous solution contains 1% acetylcholine and 3% mannitol by weight. The usual dose for administration is about 2 ml.

The second active material in the miotic composition is a compound of the formula: 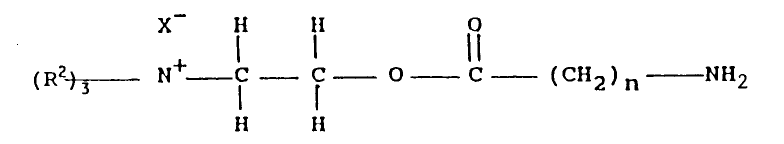 where R² is a low alkyl group of 1-5 carbon atoms, n is an integer from 0-3 and X is an anion Such as halo. The preferred material carbachol, is a compound in which n is 0, R² are all methyl and X is chloro. Carbachol is provided as a sterile aqueous salt solution in a concentration from 0.001 to 1.0 percent by weight, usually at about 0.01% by weight. The isotonic salt carrier includes the following inactive salts:

| Salt | % by Weight |
|---|---|
| NaCl | 0.64 |
| KCl | 0.075 |
| CaCl₂ · H₂O | 0.048 |
| MgCl₂ · 6H₂O | 0.03 |
| Sodium acetate · 3H₂O | 0.39 |
| Sodium citrate · 2H₂O | 0.17 |

The pH is adjusted to neutral with sodium hydroxide or hydrochloric acid as needed.

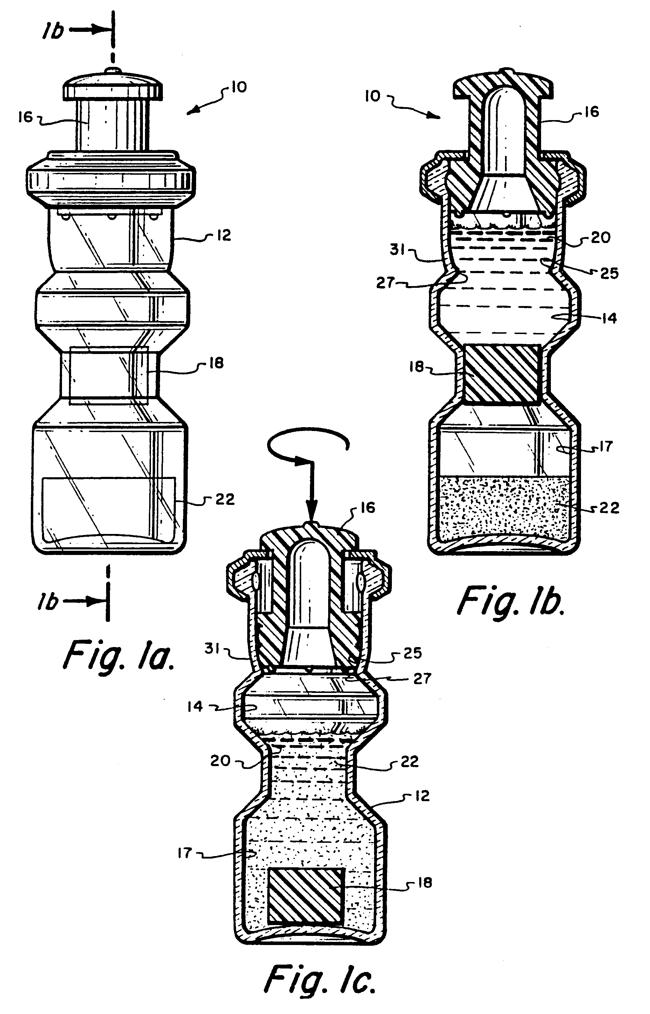

Referring now to Figure 1, the two agents can be provided in a two compartment package 10. The package is in the form of a uni-vial 12 having a first compartment 17 sealed by a first elastomer plug 18 and a second compartment 14 sealed by a second elastomer plunger-stopper 16. The first compartment 17 contains a unit dosage or charge 22 of the first agent such as acetylcholine and the lyophilizing agent such a mannitol and the second compartment 14 contains an aqueous solution 20 of the second agent in a solution of salt in isotonic proportions and concentrations. The plunger-stopper 16 is pressed downwardly which forces the plug 18 into the lower compartment. The plunger-stopper 16 comes to rest on the shoulder 25 formed along the rim 27 of the annular section 31 of the uni-vial 12. The solution 20 falls into the first compartment 17 and dissolves the dry charge 22. In use, a hypodermic needle, not shown, is inserted through the plunger-stopper 16 into the second compartment 14 to withdraw the solution. After the dry mixture of compounds is dissolved, the combined miotic agent is withdrawn into the syringe and is ready for use.

Studies were conducted using Miochol (Acetylcholine) and Miostat sequentially, and combined separately during intraocular surgical procedures.

The miotic agent of the invention can be used as follows. After extraction of a cataract, the capsule is retained. It has an anterior flap. To facilitate insertion of the lens, the capsule is inflated and Healon is applied. After insertion of the lens in the capsule, the Miochol agent is injected. As the pupil is constricting, closure of the wound takes place. The viscoelastic agent is removed from the capsule coating by aspiration with saline. The Miostat solution is then added to the anterior chamber. As the last stitching of the incision is completed, the iris is examined for roundness and for inclusion of iris or capsular tags in the incision.

When the combination of acetylcholine and carbachol is utilized in a common carrier, a portion of the combination solution is added after lens insertion and closure of the wound takes place. The viscoelastic agent is aspirated from the anterior chamber and the combination miotic agent is added again as the stitching of the wound is completed.

The following studies provide indications of the ability of the combination miotic agent of the invention to provide long-term IOP control without the use of Diamox and excellent control of glaucoma patients undergoing ECCE.

The average intraocular dose of Miochol is 0.5 to 2 ml of the 1% solution and of the Carbachol during surgery is 0.5 to 2 ml of a 0.01% solution.

The intraocular pressure was measured with the Goldman applanation tonometer the day before surgery during the pre-operative visit. The second intraocular pressure measurement was taken 20-24 hours after surgery at the first post-operative visit.

### SURGICAL TECHNIQUE

1. Standard ECCE with intraocular lens implantation (either PMMA with Prolene loops, or all PMMA) was performed. All phakoemulsification with intraocular lens implantation patients were excluded. All known glaucoma patients were also excluded from this part of the study.
2. Methods and procedures: (a) Honan cuff 15-30 minutes pre-operatively, (b) approximately 11 mm incision, (c) manual extracapsular cataract extraction (pressure below, counter-pressure above), (d) Healon (sodium hyaluronate) was used in all cases, (e) machine cortical irrigation and aspiration containing Epinephrine with either Site unit or Series 10,000 Coopervision unit, (f) "in the bag" placement of the posterior chamber intraocular lens, (g) machine irrigation and aspiration of Healon, (h) Miotic agent instilled into the anterior chamber and the chamber was aspirated to remove the Healon, (i) closure with interrupted 10-0 nylon sutures.
   Injectable medication used at surgery were Garamycin (1/2 to 1 cc), and Celestone (1/2 to 1 cc).
3. Topical medications used upon completion of surgery were Tobrex, Maxidex, and Betoptic. Celestone, Maxidex and Betoptic are underlined because they are known to affect intraocular pressure.
   ECCE surgery has been conducted utilizing acetylcholine and carbachol sequentially and combined on two sets of patients. All surgeries were successful with quick miosis followed by rapid lowering of IOP and low IOP after 24 hours.

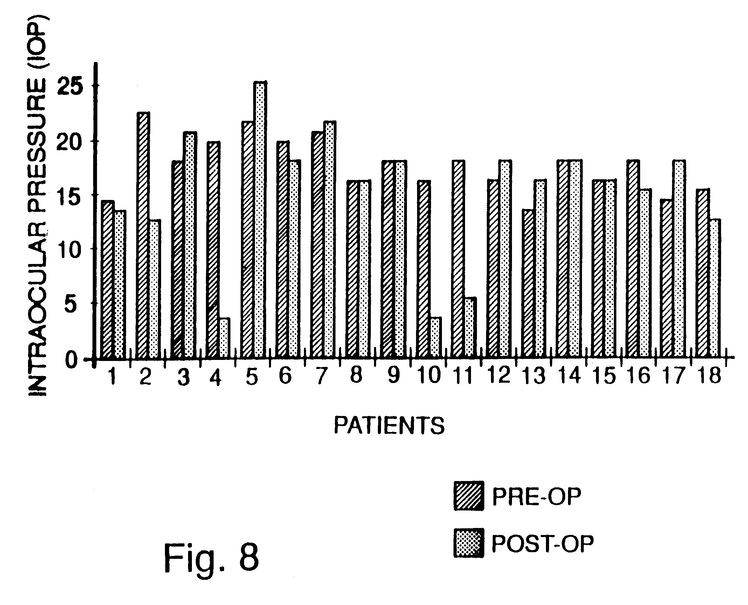

The following table and Figure 8 show the IOP of 9 patients administered the two miotic agents sequentially. Quick miosis was observed. The Post-Op IOP (24 hours after surgery) was the same or lower than the Pre-Op pressure.

**TABLE 1**

| Patient | Pre-Op | Post-Op | Delta IOP |
|---|---|---|---|
| 1 | 15 | 15 | 0 |
| 2 | 15 | 16 | 1 |
| 3 | 12 | 7 | -5 |
| 4 | 19 | 20 | 1 |
| 5 | 18 | 13 | -5 |
| 6 | 21 | 16 | -5 |
| 7 | 16 | 10 | -6 |
| 8 | 19 | 16 | -3 |
| 9 | 18 | 16 | -2 |

The combined miotic agent of the invention was administered to 18 additional subjects. Measurements of IOP were taken on the operated eye (OP) and the fellow (FEL) eye before the operation at surgery (PRE) and 24 hours after the operation (POST). The pupil size was measured at surgery (AT) and 24 hours after surgery (POST). The following Table shows the measurements, the differences (DELTA) and the maximum, minimum and averages of the measurements.

**TABLE 2**

| Patient | Pre-Op | Pre-Fel | Post-Op | Post-Fel | Pupil AT | Pupil POST | Delta OP | Delt FEL |
|---|---|---|---|---|---|---|---|---|
| 1 | 14 | 14 | 13 | 11 | 4.5 | 2 | -1 | -3 |
| 2 | 22 | 23 | 12 | 26 | 4 | 4 | -10 | 3 |
| 3 | 18 | 18 | 20 | 15 | 5 | 1.5 | 2 | -3 |
| 4 | 19 | 19 | 4 | 15 | 4 | 2.5 | -15 | -4 |
| 5 | 21 | 18 | 25 | 18 | 4 | 1.5 | 4 | 0 |
| 6 | 19 | 18 | 18 | 18 | 3 | 2 | -1 | 0 |
| 7 | 20 | 20 | 21 | 17 | 3 | 2 | 1 | -3 |
| 8 | 16 | 17 | 16 | 14 | 3.5 | 2 | 0 | -3 |
| 9 | 18 | 17 | 18 | 12 | 2.5 | 2.5 | 0 | -5 |
| 10 | 16 | 16 | 4 | 16 | 4 | 2 | -12 | 0 |
| 11 | 18 | 21 | 5 | 20 | 4 | 2 | -13 | -1 |
| 12 | 16 | 16 | 18 | 18 | 4 | 2 | 2 | 2 |
| 13 | 13 | 14 | 16 | 16 | 6 | 3 | 3 | 2 |
| 14 | 18 | 20 | 18 | 26 | 3.5 | 2 | 0 | 6 |
| 15 | 16 | 16 | 16 | 15 | 4 | 2.5 | 0 | -1 |
| 16 | 18 | 19 | 15 | 18 | 3 | 2 | -3 | -1 |
| 17 | 14 | 15 | 18 | 18 | 3.5 | 2 | 4 | 3 |
| 18 | 15 | 15 | 12 | 10 | 3 | 2 | -3 | -5 |
| Max. | 22 | 23 | 25 | 26 | 6 | 4 | 4 | 6 |
| Min. | 13 | 14 | 4 | 10 | 2.5 | 1.5 | -15 | -5 |
| Ave. | 17.278 | 17.5556 | 14.944 | 16.8333 | 3.81 | 2.194 | -2.333 | -0.72222 |
| STD | 2.4688 | 2.47867 | 5.8054 | 4.25994 | 0.82 | 0.572 | 5.9902 | 3.044871 |

The Post-Op IOP is lower than the Pre-Op IOP showing good control. The pupil is still constricted 24 hours later.

A retrospective, clinical study was conducted on pre-operative and post-operative intraocular pressures of patients administered Acetylcholine or Carbachol while undergoing extracapsular, cataract extraction with intraocular lens implantation. The surgery was performed in a similar fashion by one surgeon (the author). Intraocular pressure was measured with the Goldman applanation tonometer the day before surgery during the preoperative visit. The second intraocular pressure measurement was taken by Goldman applanation tonometer 20-24 hours following surgery at the first post-operative visit.

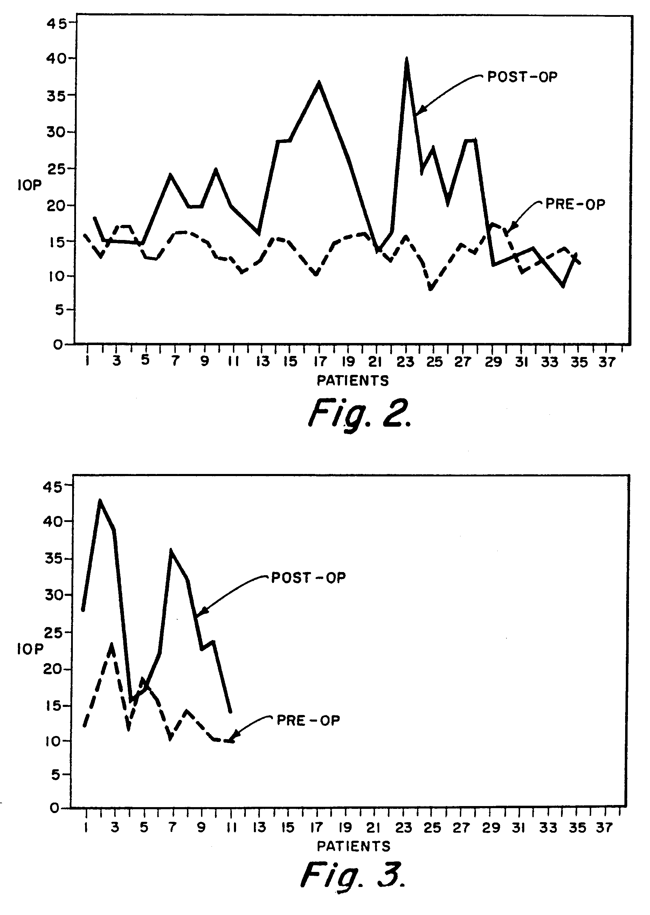

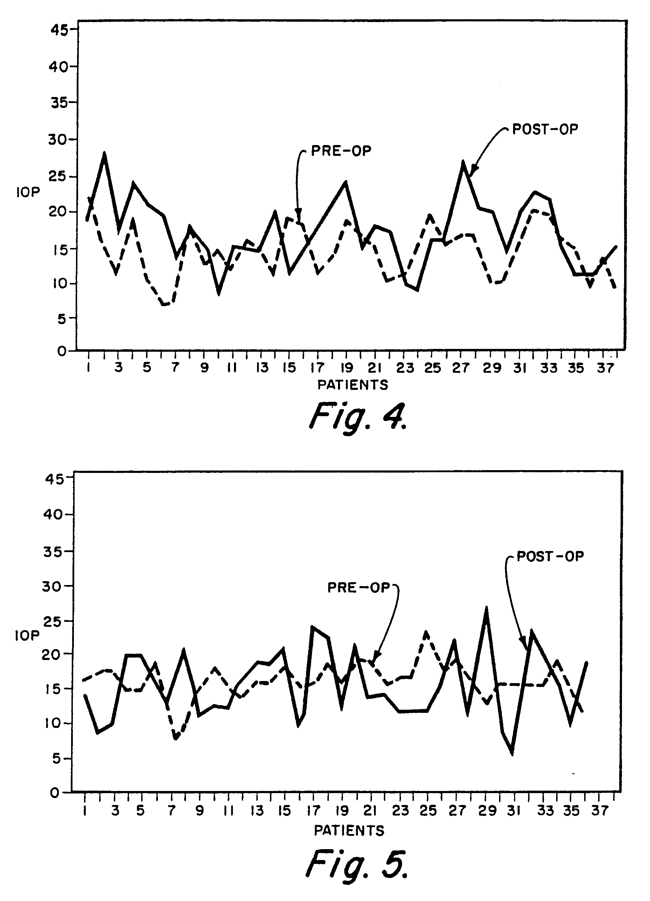

In the early stages of this retrospective study, it became apparent that post-operative pressure spikes occurring 20-24 hours later were of a major concern. This retrospective study compares the post-operative intraocular pressure (IOP) in 4 groups following standard extracapsular cataract extraction with intraocular lens (ECCE with IOL). The 4 groups consisted of Miochol (acetylcholine chloride) alone, Miochol with 500mg. Diamox (acetazolamide) given orally at 1800 hours, Miostat (Carbachol) with 500mg. Diamox given orally at 1800 hours, and fourthly, Miostat alone. Figures 2 and 3 show the spiking behavior of the group of patients treated with Miochol with or without Diamox. Figures 4 and 5 show that the patients treated with Miostat with or without Diamox had good control of IOP. Surprisingly, the group not receiving Diamox had better control.

The patients given Miochol only had an average pre-operative intraocular pressure of 14.2 and an average post-operative pressure of 26.5. However, 4 of these 11 patients had pressure spikes of over 30, and 1 of 44mmHg. The average increase in intraocular pressure was 12.3mmHG.

35 patients were given Miochol at the time of surgery with 500mg. of Diamox orally at 1800 hours on the day of surgery, in an attempt to reduce pressure spikes. The average post-operative pressure was 20.1 with an average increase of 6.8mmHg. of pressure. However, in this group, there were 9 of 35 patients with intraocular pressure of above 25 to a high as 42.

38 patients were given Miostat at the time of surgery with Diamox 500mg. at 1800 hours the day of surgery. The average post-operative intraocular pressure was 16.9, and the average increase in intraocular pressure was 2.3mm. Only 3 of 38 patients had pressures over 25, the highest being 29. This appeared to be an improvement in control of intraocular pressure 20-24 hours later over both groups of patients using Miochol.

36 patients received only Miostat at surgery with no Diamox given the day of surgery. The average post-operative intraocular pressure was reduced 0.8mm. Only 1 of 36 patients had a post-operative intraocular pressure of over 25. This group appeared to be clinically at least as well controlled as the Miostat with Diamox group, and certainly superiorly controlled to the Miochol group.

During this retrospective study, 16 known cases of glaucoma were uncovered -- an admittedly small group. All glaucoma cases were on medication and felt to be controlled, although 2 patients, 1 in each group, did have a pre-operative pressure of 25. 8 of these patients had been given Miochol at surgery, and 8 had been given Miostat. All of these patients received Diamox 500mg. at 1800 hours the day of surgery. (a) The surgical technique was the same except a full iridectomy was performed when indicted. (b) Injectable medications at the time of surgery were Garamycin and Celestone. (c) Topical medications used upon completion at surgery were Tobrex, Maxidex, and Betoptic. 500mg. of Diamox was used orally at 1800 hours the day of surgery in all cases.

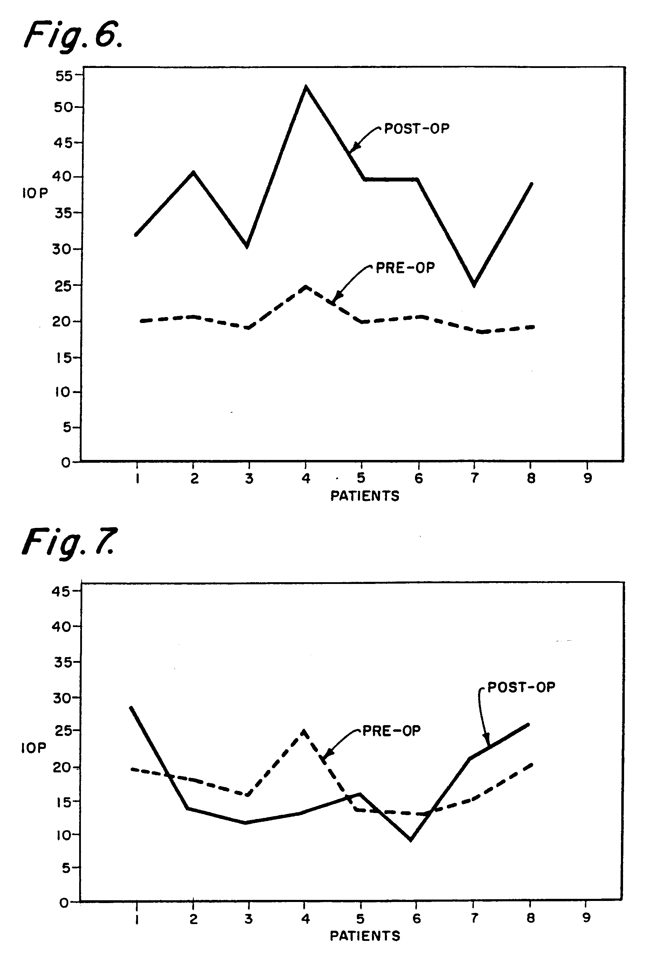

Figure 6 shows the data for the 8 patients who had been given Miochol at surgery. The average pre-operative intraocular pressure was 21, and the average post-operative intraocular pressure was 37, an increase of 16.3mm. 7 of the 8 patients had intraocular pressures of 30 or over, one patient's pressure spiked to 54mm.

Figure 7 shows the data for the 8 patients who had been given Miostat at surgery. Average pre-operative intraocular pressure was 17, and the average post-operative intraocular pressure was the same. Only 2 patients had intraocular pressures above 21, one patient of 26 and one patient of 28. A comparison of the post-operative intraocular pressures in the glaucoma population indicates that there was significant spiking of intraocular pressure 20-24 hours later in the Miochol group compared to the Miostat group of patients.

This retrospective, clinical study of the use of standard ECCE supports the safety and efficacy of the combined miotic agent of the invention. The miotic agent containing both Miochol and Miostat (carbachol .01%) appears to be a superior pharmacological agent to one containing Miochol (acetylcholine chloride 1%) in controlling IOP 20-24 hours later, as measured by applanation tonometry. In the routine care of standard ECCE with IOL, Diamox 500mg. orally at 1800 hours does not seem necessary, if using the miotic agent of the invention containing both Miochol and Miostat. In glaucoma cases, the miotic agent of the invention should be effective in providing quick miosis while controlling post-operative IOP 20-24 hours later.

It is to be realized that only preferred embodiments of the invention have been described and that numerous substitutions, modifications and alterations are permissible without departing from the scope of the invention as defined in the following claims.

## Claims

1. Product containing a first miotic agent selected frown components of the formula: 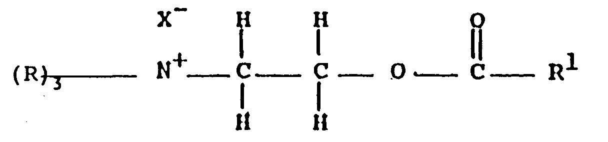 where R and R¹ are lower alkyl groups containing 1 to 5 carbon atoms and X⁻ is an anion such as halo; and
a second miotic agent selected from components of the formula: 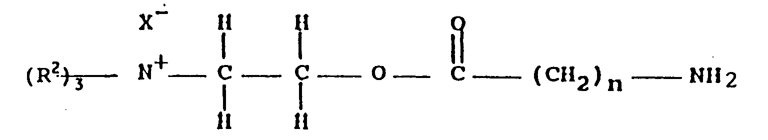 where R² is a low alkyl group of 1-5 carbon atoms, n is an integer front 0-3 and X⁻ is an anion such as halo,
as a combined preparation for simultaneous, separate or sequential use in therapy as a miotic agent controlling intraocular pressure in a mammal.

2. A product according to claim 1, in which R and R¹ are methyl and X⁻ is chloro.

3. A product according to claim 2 in which R² is methyl, n is O and X⁻ is chloro.

4. A product according to claim 3 in which the two agents are applied to the eye sequentially.

5. A product according to claim 4 in which the first agent is applied to the eye before the second agent.

6. A product according to claim 3 in which the two agents are applied to the eye simultaneously.

7. A product according to claim 3 in which the first agent is dissolved in a pharmacologically acceptable aqueous carrier in a concentration from 0.1 to 5% by weight.

8. A product according to claim 7 in which the solution of first agent further includes a lyophizing material in a weight ratio to agent of 1/1 to 10/1.

9. A product according to claim 8 in which the lyophizing material is mannitol present in an amount of about 3% by weight.

10. A product according to claim 7 in which the second agent is dissolved in a pharmacologically acceptable isotonic aqueous saline in an amount front 0.001 to 1.0 percent by weight.

11. A product according to claim 10 in which the second agent is present in the saline solution in an amount of about 0.01% by weight.

12. A product according to claim 11 in which both miotic agents are dissolved in the pharmacologically acceptable aqueous saline as a common carrier.

13. A product according to claim 12 in which the first agent is a dry powder and mannitol is a dry power in unit dosage fort which are disposed in a sterile, closed package and the second agent dissolved in said saline in unit dosage is disposed in a second sterile package and the contents of the two package to form a solution of the two agents in said saline are combined before use.

14. A product according to claim 1 in which the mammal is a human.

15. A product according to claim 14 in which the human subject is undergoing extracapsular cataract extraction with intraocular lens implant surgery.

16. A product according to claim 15 in which the human subject is suffering from glaucoma.

17. A unit dosage package (10) of a product capable of quick miosis while controlling intraocular pressure containing:
a first miotic agent selected from components of the formula: 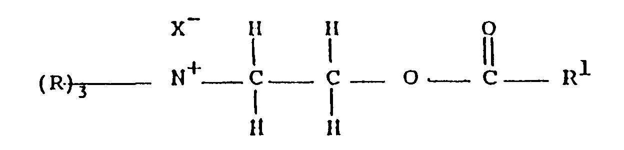 where R and R¹ are lower alkyl groups containing 1 to 5 carbon atoms aid X⁻ is an anion such as halo; and
a second miotic agent selected from components of the formula: 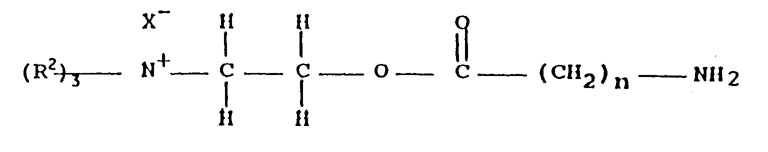 where R² is a low alkyl group of 1-5 carbon atoms, n is an integer front 0-3 and X⁻ is an anion such as halo, which comprises:
a first sterile container (17) containing a unit dosage of the first miotic agent in dry powder form and;
a second sterile container (14) containing a unit dosage of a solution of the second miotic agent in an aqueous saline which is a solvent for the first miotic agent.

18. A package according to claim 17 in which the containers are joined by an elastomer seal (16, 18) penetrable by a hypodermic needle.

19. A package according to claim 17 in which R and R¹ are methyl and X⁻ is chloro.

20. A package according to claim 19 in which R² is methyl, n is 0 and X⁻ is chloro.

## Patentansprüche

1. Produkt, welches ein erstes miotisches Mittel, ausgewählt aus Komponenten der Formel: 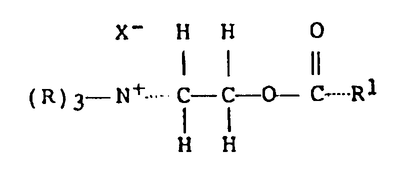 wobei R und R¹ niedere Alkylgruppen sind, welche 1 bis 5 Kohlenstoffatome enthalten, und X⁻ ein Anion wie Halogen ist; und
ein zweites miotisches Mittel enthält, ausgewählt aus Komponenten der Formel: 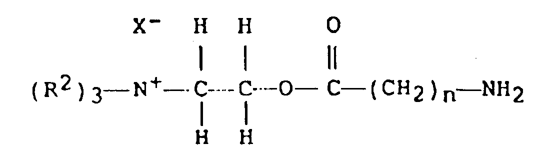 wobei R² eine niedere Alkylgruppe von 1 - 5 Kohlenstoffatomen ist, n eine ganze Zahl von 0 - 3 ist, und X⁻ ein Anion wie Halogen ist,
als ein zusammengesetztes Präparat für eine gleichzeitige, getrennte oder sequentielle Verwendung in der Behandlung als ein miotisches Mittel, welches den Augeninnendruck bei einem Säugetier reguliert.

2. Produkt gemäß Anspruch 1, in welchem R und R¹ Methyl sind, und X⁻ Chlor ist.

3. Produkt gemäß Anspruch 2, in welchem R² Methyl ist, n gleich 0 ist, und X⁻ Chlor ist.

4. Produkt gemäß Anspruch 3, in welchem die beiden Mittel dem Auge nacheinander verabreicht werden.

5. Produkt gemäß Anspruch 4, in welchem das erste Mittel dem Auge vor dem zweiten Mittel verabreicht wird.

6. Produkt gemäß Anspruch 3, in welchem die beiden Mittel dem Auge gleichzeitig verabreicht werden.

7. Produkt gemäß Anspruch 3, in welchem das erste Mittel in einem pharmakologisch geeigneten, wäßrigen Träger in einer Konzentration von 0,1 bis 5 Gew.-% gelöst ist.

8. Produkt gemäß Anspruch 7, in welchem die Lösung des ersten Mittels ferner ein lyophilisierendes (lyophizing) Material in einem Gewichtsverhältnis zu dem Mittel von 1/1 bis 10/1 enthält.

9. Produkt gemäß Anspruch 8, in welchem das lyophilisierende Material Mannitol ist, welches in einer Menge von ungefähr 3 Gew.-% vorhanden ist.

10. Produkt gemäß Anspruch 7, in welchem das zweite Mittel in einer pharmakologisch geeigneten, isotonischen, wäßrigen Salzlösung in einer Menge von 0,001 bis 1,0 Gew.-% gelöst ist.

11. Produkt gemäß Anspruch 10, in welchem das zweite Mittel in der Salzlösung in einer Menge von ungefähr 0,01 Gew.-% vorhanden ist.

12. Produkt gemäß Anspruch 11, in welchem beide miotischen Mittel in der pharmakologisch geeigneten, wäßrigen Salzlösung als einen gemeinsamen Träger gelöst sind.

13. Produkt gemäß Anspruch 12, in weichem das erste Mittel ein trockenes Pulver ist, und Mannitol ein trockenes Pulver in Form einer Dosiseinheit ist, welche in eine sterile, verschlossene Packung gefüllt sind, und das zweite Mittel, welches in der Selzlösung als Dosiseinheit gelöst ist, in eine zweite sterile Packung gefüllt ist, und der Inhalt der beiden Packungen zur Bildung einer Lösung der beiden Mittel in der Salzlösung vor der Verwendung kombiniert wird.

14. Produkt gemäß Anspruch 1, in welchem das Säugetier ein Mensch ist.

15. Produkt gemäß Anspruch 14, in welchem das menschliche Geschöpf einer extrakapsulären Starextraktion mit intraokularer Linsenimplantationsoperation unterzogen wird.

16. Produkt gemäß Anspruch 15, in welchem des menschliche Geschöpf an einem Glaukom leidet.

17. Dosiseinheitenpackung (10) eines Produktes, welches während einer Regulierung des Augeninnendrucks zu einer schnellen Pupillenverengung in der Lage ist, welches folgendes enthält:
ein erstes miotisches Mittel, ausgewählt aus Komponenten der Formel: 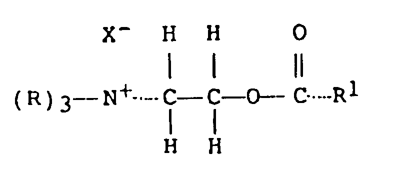 wobei R und R¹ niedere Alkylgruppen sind, welche 1 bis 5 Kohlenstoffetome enthalten, und X⁻ ein Anton wie ein Halogenanion ist; und
ein zweites miotisches Mittel, ausgewählt aus Komponenten der Formel: 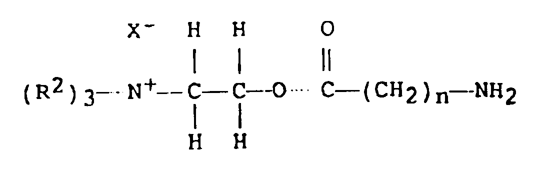 wobei R² eine niedere Alkylgruppe von 1 - 5 Kohlenstoffatomen ist, n eine ganze Zahl von 0 - 3 ist, und X⁻ ein Anion wie Halogen ist,
welches folgendes umfaßt:
einen ersten sterilen Behälter (17), welcher eine Dosiseinheit des ersten miotischen Mittels in Form eines trockenen Pulvers enthält; und
einen zweiten sterilen Behälter (14), welcher eine Dosiseinheit einer Lösung des zweiten miotischen Mittels in einer wäßrigen Salzlösung, welche ein Lösungsmittel für das erste miotische Mittel ist, enthält.

18. Packung gemäß Anspruch 17, in welcher die Behälter durch eine Elastomerdichtung (16, 18), welche von einer Injektionskanüle durchdringt werden kann, verbunden sind.

19. Packung gemäß Anspruch 17, in welcher R und R¹ Methyl sind, und X⁻ Chlor ist.

20. Packung gemäß Anspruch 19, in welcher R² Methyl ist, n gleich 0 ist, und X⁻ Chlor ist.

## Revendications

1. Produit contenant un premier agent miotique choisi parmi les composants de formule: 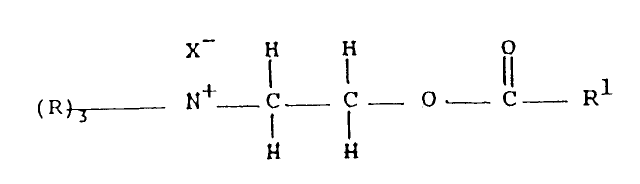 dans laquelle:
R et R¹ sont des groupes alkyle inférieurs comportant 1 à 5 atomes de carbone; et
X⁻ est un anion tel qu'un anion halogène,
et un second agent miotique choisi parmi les composants de formule: 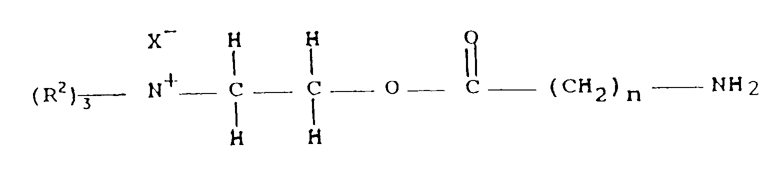 dans laquelle:
R² est un groupe alkyle inférieur comportant 1 à 5 atomes de carbone;
n est un nombre entier de 0 à 3; et
X⁻ est un anion tel qu'un anion halogène,
sous la forme d'une préparation combinée pour être utilisée simultanément, séparément ou séquentiellement en thérapie en tant qu'agent miotique pour contrôler la pression intra-occulaire chez un mammifère.

2. Un produit selon la revendication 1, dans lequel R et R¹ sont des méthyles et X⁻ est un anion chlore.

3. Un produit selon la revendication 2 dans lequel R² est un méthyle, n est égal à 0 et X⁻ est un anion chlore.

4. Un produit selon la revendication 3, dans lequel les deux agents sont appliqués à l'oeil séquentiellement.

5. Un produit selon la revendication 4, dans lequel le premier agent est appliqué à l'oeil avant le second agent.

6. Un produit selon la revendication 3, dans lequel les deux agents sont appliqués simultanément à l'oeil.

7. Un produit selon la revendication 3, dans lequel le premier agent est dissout dans un support aqueux pharmacologiquement acceptable à une concentration de 0,1 à 5 % en poids.

8. Un produit selon la revendication 7, dans lequel la solution du premier agent comprend en outre une substance lyophilisante selon un rapport pondéral par rapport à l'agent de 1/1 à 10/1.

9. Un produit selon la revendication 8, dans lequel le produit lyophilisant est du mannitol présent à raison d'environ 3 % en poids.

10. Un produit selon la revendication 7, dans lequel le second agent est dissout dans une solution saline aqueuse isotonique pharmacologiquement acceptable, à raison de 0,01 à 1,0 % en poids.

11. Un produit selon la revendication 10, dans lequel le second agent est présent dans la solution saline à raison d'environ 0,01 % en poids.

12. Un produit selon la revendication 11, dans lequel les deux agents miotiques sont dissous dans la solution aqueuse pharmacologiquement acceptable servant de support commun.

13. Un produit selon la revendication 12, dans lequel le premier agent est une poudre sèche et le mannitol est une poudre sèche, sous forme galénique unitaire, et sont placés dans un emballage fermé, stérile et le second agent est dissout dans ladite solution saline sous forme galénique unitaire placée dans un second emballage stérile et en ce que l'on combine le contenu des deux emballages pour former une solution des deux agents dans ladite solution saline avant l'emploi.

14. Un produit selon la revendication 1, dans lequel le mammifère est l'Homme.

15. Un produit selon la revendication 14, dans lequel le patient est en train de subir une extraction de la cataracte extracapsulaire avec chirurgie d'implantation d'une lentille intraocculaire.

16. Un produit selon la revendication 15, dans lequel le patient souffre de glaucome.

17. Un emballage galénique unitaire (10) d'un produit capable de provoquer une miosis rapide tout en contrôlant la pression intra-occulaire, comprenant:
- un premier agent miotique choisi parmi les composants de formule: 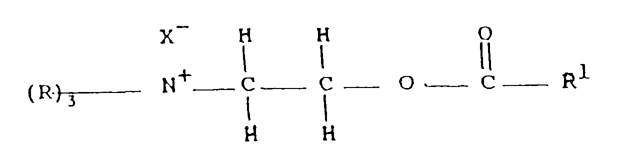
dans laquelle:
R et R¹ sont des groupes alkyle inférieurs comportant 1 à 5 atomes de carbone; et
X⁻ est un anion tel qu'un anion halogène,
et
- un second agent miotique choisi parmi les composants de formule: 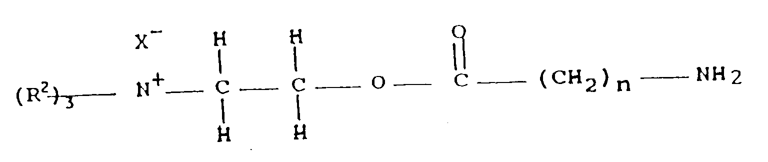
dans laquelle:
R² est un alkyle inférieur comportant 1 à 5 atomes de carbone;
n est un nombre entier de 0 à 3; et
X⁻ est un anion tel qu'un anion halogène,
qui comprend:
- un premier emballage stérile (17) contenant une préparation galénique unitaire du premier agent miotique sous forme de poudre sèche, et
- un second emballage stérile (14) contenant une préparation galénique unitaire d'une solution du second agent miotique dans une solution saline aqueuse qui est un solvant du premier agent miotique.

18. Un emballage selon la revendication 17, dans lequel les emballages sont réunis par un joint en élastomère (16, 18) tranperçable par une aiguille hypodermique.

19. Un emballage selon la revendication 17, dans lequel R et R¹ sont des méthyles et X⁻ est un anion chlore.

20. Un emballage selon la revendication 19, dans lequel R² est un méthyle, n est zéro et X⁻ est un anion chlore.
